(19) Europäisches Patentamt

European Patent Office

Office européen des brevets

(11) **EP 3 669 921 A1**

(12) **EUROPEAN PATENT APPLICATION**

(43) Date of publication:
**24.06.2020 Bulletin 2020/26**

(51) Int Cl.:
*A61M 21/00* (2006.01)   *A61M 21/02* (2006.01)

(21) Application number: **18212883.5**

(22) Date of filing: **17.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **VAN DEN ENDE, Daan Anton**
  **5656 AE Eindhoven (NL)**
- **PASTOOR, Sander Theodoor**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A SYSTEM AND METHOD FOR DELIVERING AUDITORY SLEEP STIMULATION**

(57)     A system delivers auditory sleep stimulation. It is able to generate masking sounds for masking external noise as well as sleep stimulation tones for promoting deep sleep. Noise masking sounds are provided in a frequency range including first and second frequency bands, before the onset of sleep. In response to the detection of particular sleep characteristics (such as a particular sleep stage), the noise masking sounds in the second frequency band are stopped. Instead, sleep stimulation tones are provided in the second frequency band. Thus, noise masking continues but the sleep stimulation tones are not masked.

FIG. 1

**Description**

FIELD OF THE INVENTION

The present invention relates to the field of audio systems, and in particular to audio systems used for influencing a sleep state of a subject.

BACKGROUND OF THE INVENTION

Recent research has shown that auditory stimulation applied during sleep can provide cognitive benefits and enhancements of sleep restoration for a subject or user. It has also been recognized that appropriately controlled audio outputs can help influence a sleep state of the subject, so as to influence at least whether the subject is awake or asleep.

There are numerous different causes for disturbance during sleep, external to the subject (e.g. traffic noise, aircraft, snoring partners, neighbors, construction noise, insect noise, electrical appliances etc.) and internal. The internal causes include physiological (e.g. tinnitus), psychological (e.g. stress) and behavioral (e.g. poor sleeping practice) causes.

It has been recognized that playing audio can lead to sleep quality improvement, especially by improving the ability initially to fall asleep in the evening, and also after waking up in the middle of the night.

The external disturbances can be alleviated by playing a masking sound or by using anti-noise (i.e. a sound cancellation system). A masking sound typically is a recorded repetitive sound (such as rain or ocean waves) or a generated random waveform with equally distributed acoustic intensity over the audible frequency range (termed 'white noise'). These sounds all aim to drown out sudden and/or annoying external noise and can be clustered under the term 'masking sound'.

Anti-noise (sound cancellation) is a special form of masking sound which needs a microphone close to the ear to pick up the sound vibrations in order to play the right phase-shifted anti-noise. Sleep compatible noise cancellation headphones have been proposed.

The major cause of internal disturbance is typically stress or worrying. This can be solved by playing calming sounds or music, guided meditation and/or randomly generated words which all aim to reduce the state of arousal of the mind. These methods all aim to calm the user down so they can go to sleep more easily and can be clustered under the term 'calming audio'. Often, a combination of calming audio and background music is used.

A system known as "SmartSleep" of the applicant uses a sleep detection feature to create a feedback loop. An electroencephalogram (EEG) signal analysis is used to detect deep sleep in real-time and the system delivers auditory stimulation (sleep enhancing tones) to enhance sleep slow waves without causing arousals. In addition to deep sleep, other sleep stages (e.g. an awake state) can be detected from the EEG signal as well as a depth of sleep.

WO 2018/001936 discloses a system for adjusting a volume of sleep stimulation tones, in dependence on a depth of sleep, within this type of system. Thus, it is known to control the sleep stimulation tones using feedback from sleep monitoring.

However, current masking sound or calming audio solutions are without feedback, so that the users have the choice to play the audio all night long or for a predetermined time. In many cases, such a 'deadline' for falling asleep is counterproductive for users with problems falling asleep.

Furthermore, the combination of masking sounds (or calming audio) with sleep enhancing tones is difficult to implement, because the masking sounds (or calming audio) undermine the effectiveness of the sleep enhancing tones.

There is therefore a need for a system which can combine the benefits of assisting a user to fall or stay asleep as well as providing different sleep enhancing tones during sleep.

SUMMARY OF THE INVENTION

The invention is defined by the claims.

According to examples in accordance with an aspect of the invention, there is provided a system for delivering auditory sleep stimulation, comprising:

a sound generator system for generating masking sounds for masking external noise and sleep stimulation tones;
a sleep monitor; and
a controller, wherein the controller is adapted to:

use the sound generator system to provide noise masking sounds in a frequency range including first and second frequency bands, before the onset of sleep;
use the sleep monitor to detect particular sleep characteristics;
in response to the detection of the particular sleep characteristics, stop providing the noise masking sounds in the second frequency band; and
provide sleep stimulation tones in the second frequency band.

This system enables masking sounds to be used while a subject is going to sleep. The masking sounds may be for noise cancelling or for drowning out noise, and both of these possibilities are intended to be included in the term "masking". However, instead of turning off the masking sounds at a certain time, or maintaining them all the time, a sub-band of the frequency spectrum is turned off when particular sleep characteristics are detected. This particular sleep characteristics might simply be the onset of sleep, but they might be any particular sleep stage, depth of sleep or combination of sleep stage and depth of sleep.

This means that the masking function (outside of the second frequency band) may remain active during the night so that the risk of external noises awakening the subject are reduced. However, the effectiveness of sleep stimulation tones is preserved, since they fall within that sub-band of the frequency spectrum. The invention thus combines the advantages of noise masking and sleep stimulation through the sleep period.

The controller may be adapted to stop providing the noise masking sounds by fading them out. This provides a less noticeable change which is therefore less likely to disturb the user's sleep.

The controller may be adapted to provide sleep stimulation tones during all detected stages of sleep. Thus, they may remain active during sleep, but the characteristics may be adjusted, such as frequency and/or amplitude. Alternatively, they may be used only during certain sleep stages.

The sleep stimulation tones for example comprise a sequence of temporally separated pulses. It is known to use such pulses to increase slow wave activity (SWA) and thereby prolong deep sleep.

The masking sounds may comprise white noise. These are known to provide masking of external sounds.

The second frequency band may for example have a lowest value in the range 400Hz to 600Hz and a highest value in the range 1500Hz to 2500Hz, for example the second frequency band comprises 500Hz to 2kHz. This is a known frequency band for sleep stimulation tones.

The first frequency band for example has a first sub-band immediately below the lowest value and a second sub-band immediately above the highest value. In this way, the first and second bands together define a continuous overall frequency band.

The sleep monitor may be for detecting a sleep stage and/or a depth of sleep. The particular sleep characteristics, which is used to trigger when to stop masking in the frequency band of the sleep stimulation tones, may thus be a combination of a sleep stage and a depth of sleep.

The sleep monitor for example comprises an EEG monitor. EEG monitoring is known for sleep stage detection and analysis, and is suitable for determining when the sleep stimulation tones should be presented without disturbance by masking sounds.

The controller may be further adapted to provide pink noise during some or all detected stages of sleep.

Pink noise may be defined as noise with a power spectral density of the form $S(f) \propto 1/f^{\alpha}$

where f is frequency, and $0 < \alpha < 2$, with exponent $\alpha$ usually close to 1.

Pink-like noises occur widely in nature and are a source of considerable interest in many fields. They have been found to be of interest for deep sleep stimulation.

The invention also provides a method for delivering auditory sleep stimulation, comprising:

generating masking sounds for masking external noise in a frequency range including first and second frequency bands before the onset of sleep;
monitoring sleep, and in response to detection of particular sleep characteristics, stopping the provision of the noise masking sounds in the second frequency band; and
providing sleep stimulation tones in the second frequency band.

This method suppresses masking sounds in the frequency band used by sleep stimulation tones. The method may comprise stopping the provision of the noise masking sounds by fading them out.

The second frequency band for example has a lowest value in the range 400Hz to 600Hz and a highest value in the range 1500Hz to 2500Hz, for example the second frequency band comprises 500Hz to 2kHz. The first frequency band for example has a first sub-band immediately below the lowest value and a second sub-band immediately above the highest value.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a system for controlling the delivery of auditory stimulation to a subject during sleep; and
Fig. 2 shows a method for controlling the delivery of auditory stimulation to a subject 12 during sleep.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system which delivers auditory sleep stimulation. It is able to generate masking sounds for masking external noise as well as sleep stimulation tones for promoting deep sleep. Noise masking sounds are provided in a frequency range including first and second frequency bands, before the onset of sleep. In response to the detection of particular characteristics, such as a particular sleep stage, the noise masking sounds in the second frequency band are stopped. Instead, sleep stimulation tones are provided in the second frequency band. Thus, noise masking continues but the sleep stimulation tones are not masked.

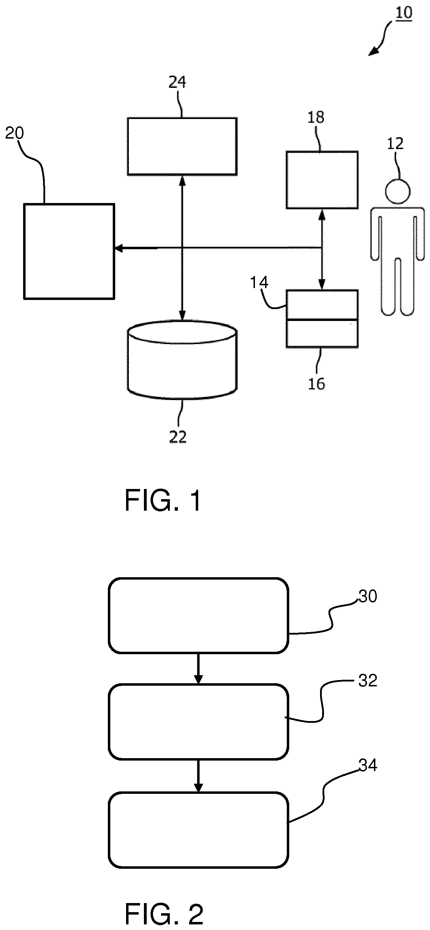

Fig. 1 is a schematic illustration of a system 10 configured to control the delivery of auditory stimulation to a subject 12 during sleep. The restorative value of sleep may be enhanced by increasing slow wave activity (SWA) in subject 12 using auditory stimulation during sleep. In addition, masking sounds may be used to assist the subject in falling asleep, either initially or after waking in the night.

The system comprises a sound generator system 14, 16. The system comprises a first sound generator 14 for generating masking sounds for masking external noise and a second sound generator 16 for generating sleep stimulation tones. Of course, in practice there may be a single hardware unit for generating all the different sounds and a single sound delivery system. The functionality is shown separately in Fig. 1 for the purposes of explanation only.

A sleep monitor 18 is used for monitoring sleep characteristics, and in particular for detecting a sleep stage and preferably also a depth of sleeping.

A controller 20 controls the sound generators 14,16 in response to particular detected sleep characteristics, such as sleep stage and/or depth.

In particular, the first sound generator 14 is used to provide noise masking sounds in a frequency range including first and second frequency bands, before the onset of sleep. This may together define a continuous frequency covering the full audible spectrum, i.e. including the range 20Hz to 20kHz.

Within this overall frequency band, a first frequency band has two sub-bands; a first below 500Hz and a second above 2kHz. A second frequency band is the range 500Hz to 2kHz.

Systems are known which generate sleep stimulation tones which are randomly distributed between 500Hz and 2kHz frequencies. The frequency bands outside of this range can be source of disturbing noise, if they are not masked. For instance, there may be aircraft noise at around 100Hz, snoring at around 400Hz, alarms at around 4kHz and insect sounds at around 10kHz. These all fall outside of the 500Hz - 2kHz range.

The invention involves stopping, and preferably fading out, the frequency band used for the sleep stimulation tones from the masking sounds. The signal generated for masking purposes, such as white noise signal, is filtered to remove those frequencies. The masking sounds may for example comprise any noise signal, which thus provides a noise floor across a broad frequency spectrum. In this way, sounds of high (>2kHz) and low (<500Hz) frequency can be masked during all stages of sleep, without affecting the ability for the subject to register the sleep stimulating tones.

The noise masking sounds are thus unfiltered when they include both frequency bands, i.e. a continuous spectrum, and they are filtered when the second frequency band is removed.

When the subject initially intends to sleep, the unfiltered noise masking sound is provided across the full frequency spectrum. During this time, the sleep stimulation tones may be provided as well (even though they are intended for use during deep sleep) or else they may not yet be started.

In response to the detection of the particular sleep characteristics, the noise masking sounds are suppressed in the second frequency band. The sleep stimulation tones are then provided in the second frequency band. They may already have been provided or else they may be started in response to the detection of the particular sleep characteristics.

The filtered masking sounds may be kept active during all sleep stages, including during deep sleep. However, the filtered masking sounds may be provided only for a subset of the sleep stages or indeed only for particular depths of sleep.

The sleep stimulation tones, when provided during sleep, enhance sleep slow waves and increase the restorative value of sleep.

The sleep monitor 18 comprises electroencephalogram (EEG) system for monitoring brain wave activity.

The system further comprises electronic storage 22 and a user interface 24. While the sound generators 14,16, sleep monitor 18, processor 20, electronic storage 22, and user interface 24 are shown as separate entities, this is not intended to be limiting. Some and/or all of the components of system 10 and/or other components may be grouped into one or more singular devices. For example, some and/or all of the components of system 10 may be grouped as part of a headband and/or other garments worn by the subject 12.

The sound generators 14,16 provide auditory stimuli to subject 12 prior to a sleep session, during a sleep session, after a sleep session, and/or at other times. The sounds are intended to induce, maintain, and/or adjust slow wave activity, for example as indicated by EEG power in the 0.5 to 4 Hz band in subject 12.

The delivery of the sleep stimulation tones is timed at least to correspond to sleep stages associated with SWA, but they may be provided at other times. The system may also be used to generate sounds to wake the subject 12 from sleep.

The invention makes use of sound generation, but this does not exclude use of other stimuli such as odors, visual stimulation, touch, taste, and/or other stimuli. For example, transcranial magnetic stimulation may be applied to subject 12 to trigger, increase, and/or decrease SWA.

The sleep monitor 18 is used to generate output signals conveying information related to brain activity of subject 12. The sleep monitoring takes place during a sleep session of subject 12, at regular intervals during a sleep session, before a sleep session, after a sleep session, and/or at other times. The brain activity of subject 12 may correspond to sleep depth, a current sleep stage, SWA in subject 12, and/or other characteristics of subject 12.

The sleep monitor comprises EEG electrodes although other sensors may be used. An EEG signal exhibits changes throughout a sleep session. A prominent change in the EEG delta power (corresponding to SWA) is typically visible, for example. SWA corresponds to the power of an EEG signal in the 0.5-4 Hz band (or a 0.5-4.5 Hz band). SWA has a typical behavior throughout cyclic variations of a given sleep session.

Different sleep stages are associated with different brain activity characteristics, which may be sensed by the sleep monitor. In particular, the brain activity of subject 12 may be associated with rapid eye movement (REM) sleep, non-rapid eye movement (non-REM) sleep, and/or other sleep. Sleep stages of subject 12 may include one or more of non-REM stage N1, stage N2, or stage N3 sleep, REM sleep, and/or other sleep stages. N1 corresponds to a light sleep state and N3 corresponds to a deep sleep state. Non-REM stage N3 or stage N2 sleep may be slow wave (e.g., deep) sleep.

SWA increases during non-rapid eye movement sleep, declines before the onset of rapid-eye-movement sleep, and remains low during REM sleep. SWA in successive non-REM episodes progressively decreases from one episode to the next. SWA may be estimated, and/or slow wave sleep (e.g., stage N3) may be determined from an EEG for subject 12 during a given sleep session.

By way of example, in the system of the invention, the unfiltered masking noise may be used in wakefulness and in sleep during the REM sleep stage and in non-REM sleep during stages N1 and N2. Sleep stimulation tones are used during deep sleep, during the N3 stage of non-REM sleep. Thus, the particular characteristics of the sleep used to determine when to perform filtering may comprise the non-REM sleep stage N3.

The unfiltered masking noise in the second frequency band may be gradually filtered out during the start of an episode of sleep stage N3. The filtering function may be increased during a certain period after the detection of the beginning of an N3 sleep stage episode, for instance 60 seconds, after which the second frequency band is fully filtered out of the masking noise. Subsequently the sleep stimulation tones begin to be used.

The gradual filtering function may be implemented over a different time period, for example generally between 15 second and 5 minutes. The attenuation provided by the filtering may be increased linearly over that time, but equally other time-dependent functions may be used.

Other sensor data may be used such as a heart rate of subject 12 (e.g. using a heart rate sensor located on the chest of subject 12, or a bracelet on a wrist of subject 12), movement of subject 12 (e.g. using an accelerometer sensor system), respiration of subject 12, and/or other characteristics of subject 12.

Although the sleep monitor 18 is illustrated at a single location near the subject 12, this is not intended to be limiting. The sleep monitor 18 may include sensors disposed in a plurality of locations, such as for example, coupled in a removable manner with the skin of subject 12, coupled in a removable manner with clothing of subject 12, or worn by subject 12 (e.g., as a headband, wristband, etc.) or not coupled to the subject, such as an accelerometer system coupled to the bed of the subject 12 or a video camera viewing subject 12.

The controller 20 provides information processing capabilities in the system 10. As such, processor 20 may comprise one or more of a digital processor, an analog processor, a digital circuit designed to process information, an analog circuit designed to process information, a state machine, and/or other mechanisms for electronically processing information.

The controller 20 may comprise one or more processing units, which may be at a single location or distributed. It is used to analyze the EEG signals, for example to determine the power in various frequency bands of the EEG, ratios of power in a high (e.g., alpha, beta) frequency band to power in a low (e.g., delta, theta) frequency band, and/or other parameters.

Brain activity parameters are related to a frequency, amplitude, phase, and/or presence of specific sleep patterns such as spindles, K-complexes, or sleep slow waves, alpha waves, and/or other characteristics of the EEG signal. For example, individual slow waves may be identified by detecting a negative going zero-crossing followed by a positive going zero-crossing in an EEG signal where the amplitude of the negative peak is below a first predetermined threshold and the time period between the zero crossings is longer than a second predetermined threshold (e.g., 200 milliseconds).

A sleep depth may be identified as well as a sleep state. This may for example involve determining (i) a ratio of power in a high frequency band of an EEG signal to power in a low frequency band, (ii) a density of slow waves in subject 12, (iii) a hypnogram for the first sleep session, a frequency of spindles in subject 12, and/or other parameters indicative of sleep depth in subject 12. For example a power ratio between the alpha (8-12Hz) or beta (15-30Hz) bands, and the delta (0.5-4Hz) or theta (4-8Hz) bands of the EEG signal are of interest.

The system may also detect the presence of sleep arousal events. Arousal events are also detected using thresholds on the EEG power in the higher alpha (8-12Hz) and beta (15-30Hz) bands. If an arousal is detected during stimulation, the stimulation may then be stopped immediately. If an arousal is detected outside the stimulation period, the onset of the next stimulation segment may be delayed.

If no arousal is detected, a typical system proceeds to detect, in real-time, the presence of slow wave sleep (e.g., N3 sleep), which is characterized by high activity in the lower delta frequency range (0.5 to 4 Hz) of the EEG. If slow wave sleep is detected for a sufficiently long period of time, then a typical system delivers auditory stimulation which for example consists of a sequence of 50-millisecond long tones separated from each other by a constant 1 second long inter-tone interval. Alternatively, the tones may be separated by a variable time interval, for example a random time interval. The 50ms time period is also only one possible example.

The sound in the 50ms periods may be sine wave (i.e. single frequency) tones, but equally white noise or pink noise may be used.

WO 2015/049613 discloses various options for the stimuli provided to the subject to provide sleep stimulation.

The volume of the auditory stimulation may be progressively increased to enhance slow waves in the user. There are known ways to implement control of the volume of the stimulation for example as disclosed in WO 2018/001936.

It is described above how the system alters the masking sounds over time in dependence on the sleep characteristics, i.e., sleep stage and/or depth, of the subject.

Additional optional adaptations to the system functionality are discussed below.

The pulsed sleep stimulating tones are for example randomly distributed between 500Hz and 2kHz frequency as explained above. It has been reported that pulses of pink noise may be delivered when the upstate of the slow wave was predicted, to increase slow wave activity (SWA). Reference is made to Papalambros NA, Santostasi G, Malkani RG, Braun R, Weintraub S, Paller KA and Zee PC (2017) Acoustic Enhancement of Sleep Slow Oscillations and Concomitant Memory Improvement in Older Adults. Front. Hum. Neurosci. 11:109. doi: 10.3389/fnhum.2017.00109.

When pink noise is output during the entire night, specific volume peaks can be used to stimulate slow waves. Effectively the constant pink noise increases the noise floor for the stimulation tones and the volume peaks rising above the constant noise floor effectively act as tones to stimulate slow wave enhancement.

The volume distribution (with respect to frequency) is dictated by the 1/f condition, but the total volume (amplitude) may be varied to create volume peaks at different times.

Masking sounds are discussed above. However, the system may also provide other sleep induction sounds, such as music, random words etc. as discussed above.

As in known devices, the sounds used for sleep induction (masking sound or calming audio) may be slowly reduced when sleep sets in. The masking sound volume may be controlled to be inversely proportional to the sleep depth as measured from the EEG traces of the subject.

As mentioned above, the sleep depth is defined as the ratio of the EEG power in the beta band to the EEG power in the delta band. The generic algorithm for volume of the masking sound may then be given by:

$$P_{set} = P_{init} \left( \frac{SD_{threshold} - SD}{SD_{threshold}} \right)$$

Where Pset is the sound power set point, Pinit is the sound power at the set volume by the user at the start of the session and SDthreshold is the sleep depth threshold at which the algorithm is activated. SD is the time averaged sleep depth (for instance over the previous 30 seconds).

The update frequency of the set point can be fixed (for instance 30 seconds) and the sound output power will transition linearly towards the new set point value.

In addition to the sleep depth, the slow wave density and delta power are also possible candidates to replace SD in the above formula. Theta power may also be used as a valid measure, due to the close relationship between cardiac autonomic function and activity of medial frontal neural circuitry.

Similarly, when waking is detected after sleep onset by the system, the masking sound can be resumed to allow the user to fall back to sleep more easily by blocking external sounds. Alternatively, the user may opt for a different kind of audio for instance changing from masking to calming or from spoken guided meditation before initial sleep onset to calming nature sounds after an awakening during the night.

Similarly, when light sleep is detected after a period of deep sleep by the system, the masking sound can be resumed, since no stimulation tones are provided during light sleep. This can allow the user to remain in light sleep more easily by blocking external sounds that could otherwise wake the user up. Alternatively, the user my opt for a different kind of audio, for instance changing from masking to calming or from spoken guided meditation before initial sleep onset to calming nature sounds or white noise when light sleep is detected during the night.

In general, the system starts playing masking sounds (and optionally calming audio or a combination of the two) before going to sleep while simultaneously monitoring the sleep stage and depth of sleep. Volume and tone (frequency) as well as the type of sound may controlled in dependence on the different sleep stages.

Fig. 2 shows a method for delivering auditory sleep stimulation, comprising:

in step 30, generating masking sounds for masking external noise in a frequency range including first and second frequency bands before the onset of sleep;

in step 32, detecting particular sleep characteristics, and in response thereto stopping the provision of the noise masking sounds in the second frequency band; and

in step 34 providing sleep stimulation tones in the second frequency band.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A system for delivering auditory sleep stimulation, comprising:

a sound generator system (14,16) for generating masking sounds for masking external noise and for generating sleep stimulation tones;
a sleep monitor (18); and
a controller (20), wherein the controller is adapted to:

use the sound generator system (14,16) to provide noise masking sounds in a frequency range including first and second frequency bands, before the onset of sleep;
use the sleep monitor (18) to detect particular sleep characteristics;
in response to the detection of the particular sleep characteristics, stop providing the noise masking sounds in the second frequency band; and
provide sleep stimulation tones in the second frequency band.

2. A system as claimed in claim 1, wherein the controller (20) is adapted to stop providing the noise masking sounds by fading them out.

3. A system as claimed in claim 1 or 2, wherein the controller (20) is adapted to provide sleep stimulation tones during all detected stages of sleep.

4. A system as claimed in any one of claims 1 to 3, wherein the sleep stimulation tones comprise a sequence of temporally separated pulses.

5. A system as claimed in any one of claims 1 to 4, wherein the masking sounds comprise white noise.

6. A system as claimed in any one of claims 1 to 5, wherein the second frequency band has a lowest value in the range 400Hz to 600Hz and a highest value in the range 1500Hz to 2500Hz, for example the second frequency band comprises 500Hz to 2kHz.

7. A system as claimed in claim 6, wherein the first frequency band has a first sub-band immediately below the lowest value and a second sub-band immediately above the highest value.

8. A system as claimed in any one of claims 1 to 7, wherein the sleep monitor (18) is for detecting sleep stages and/or a depth of sleep.

9. A system as claimed in any one of claims 1 to 8, wherein the sleep monitor (18) comprises an EEG monitor.

10. A system as claimed in any one of claims 1 to 9, wherein the controller (20) is further adapted to provide pink noise during some or all detected stages of sleep.

11. A method for delivering auditory sleep stimulation, comprising:

(30) generating masking sounds for masking external noise in a frequency range including first and second frequency bands before the onset of sleep;
(32) monitoring sleep, and in response to detection of particular sleep characteristics, stopping the provision of the noise masking sounds in the second frequency band; and
(34) providing sleep stimulation tones in the second frequency band.

12. A method as claimed in claim 11, comprising stopping the provision of the noise masking sounds by fading them out.

13. A method as claimed in claim 11 or 12, wherein the second frequency band has a lowest value in the range 400Hz to 600Hz and a highest value in the range 1500Hz to 2500Hz, for example the second frequency band comprises 500Hz to 2kHz.

14. A method as claimed in claim 13, wherein the first frequency band has a first sub-band immediately below the lowest value and a second sub-band immediately above the highest value.

15. A method as claimed in any one of claims 11 to 14, comprising providing pink noise during all detected stages of sleep.

FIG. 1

FIG. 2

Europäisches
Patentamt

European
Patent Office

Office européen
des brevets

# EUROPEAN SEARCH REPORT

Application Number

EP 18 21 2883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 886 707 A1 (FUTURE ACOUSTIC LLP [GB]) 13 February 2008 (2008-02-13) * abstract; figures 1-9 * * paragraphs [0043] - [0046], [0056], [0057] * | 1-15 | INV. A61M21/00 A61M21/02 |
| A | WO 2014/200433 A1 (AGENCY SCIENCE TECH & RES [SG]) 18 December 2014 (2014-12-18) * abstract; figures 1-7 * | 1-15 | |
| A | WO 2015/118415 A1 (KONINKL PHILIPS NV [NL]; WISCONSIN ALUMNI RES FOUND [US]) 13 August 2015 (2015-08-13) * abstract; figures 1-11 * | 1-15 | |

TECHNICAL FIELDS
SEARCHED        (IPC)

A61M

The present search report has been drawn up for all claims

| Place of search | Date of completion of the search | Examiner |
|---|---|---|
| Munich | 29 May 2019 | Weijland, Albert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 21 2883

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2019

| Patent document cited in search report | | Publication date | Patent family member(s) | | Publication date |
|---|---|---|---|---|---|
| EP 1886707 | A1 | 13-02-2008 | NONE | | |
| WO 2014200433 | A1 | 18-12-2014 | CN | 105451801 A | 30-03-2016 |
| | | | EP | 3007755 A1 | 20-04-2016 |
| | | | SG | 11201510213U A | 28-01-2016 |
| | | | US | 2016151602 A1 | 02-06-2016 |
| | | | WO | 2014200433 A1 | 18-12-2014 |
| WO 2015118415 | A1 | 13-08-2015 | CN | 105960195 A | 21-09-2016 |
| | | | EP | 3102094 A1 | 14-12-2016 |
| | | | JP | 6499189 B2 | 10-04-2019 |
| | | | JP | 2017509374 A | 06-04-2017 |
| | | | US | 2017000970 A1 | 05-01-2017 |
| | | | WO | 2015118415 A1 | 13-08-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018001936 A

- WO 2015049613 A

**Non-patent literature cited in the description**

- **PAPALAMBROS NA ; SANTOSTASI G ; MALKANI RG ; BRAUN R ; WEINTRAUB S ; PALLER KA ; ZEE PC.** Acoustic Enhancement of Sleep Slow Oscillations and Concomitant Memory Improvement in Older Adults. *Front. Hum. Neurosci,* 2017, vol. 11, 109